# EUROPEAN PATENT APPLICATION

(11) **EP 2 926 662 A1**
(43) Date of publication of application: **07.10.2015**
(21) Application number: 13848947.1
(22) Date of filing: 22.10.2013
(51) Int. Cl.: A01N 55/00, A01N 25/00, A01N 25/02, A01N 25/22, A01P 1/00, A01P 3/00, A61K 31/695, A61P 31/04, A61P 31/10, A61P 31/12, D06M 13/513, D06M 15/11, A01N 33/12

(54) **SILICON-CONTAINING COMPOUND AQUEOUS SOLUTION AND ANTIBACTERIAL/ANTIVIRAL AGENT INCLUDING SAID SOLUTION**

(30) Priority: 22.10.2012 JP 2012233292
(71) Applicant: Jex Co., Ltd., Osaka 540-0012 (JP); Hiroshima University, Higashi-Hiroshima-shi Hiroshima 739-8511 (JP)
(72) Inventor: NAGAI Satoshi, Osaka-shi Osaka 540-0012 (JP); RIKO Katsuhisa, Osaka-shi Osaka 540-0012 (JP); NIKAWA, Hiroki, Hiroshima-shi Hiroshima 734-8553 (JP)
(74) Representative: Jostarndt Patentanwalts-AG
(86) International application number: PCT/JP2013/078624
(87) International publication number: WO 2014/065297

(57) **Abstract**

[Object] The present invention provides an antibacterial and antiviral agent that includes an aqueous solution of a silicon-containing compound that has an antibacterial functional group, the aqueous solution having high stability over time and a strong deodorant effect.

[Means of Realizing the Object] An aqueous solution that includes a silicon-containing compound represented by the following general formula (1) 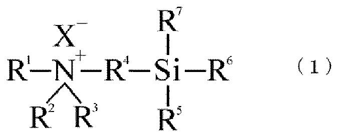 (in which, R1 represents a hydrocarbon group having 6 or more carbon atoms, R2 and R3 are optionally the same or different and represent a lower hydrocarbon group, R4 represents a divalent lower hydrocarbon group, R5, R6, and R7 are optionally the same or different and represent a lower alkyl group or a lower alkoxy group, and X represents a halogen ion or an organic carbonyloxy ion), and a cyclodextrin or a cyclodextrin derivative

## Description

### Technical Field

The present invention related to aqueous solutions of silicon-containing compounds and antibacterial and antiviral agents that include the aqueous solutions. In particular, the present invention relates to an antibacterial and antiviral agent that includes an aqueous solution of a silicon-containing compound, which has an antibacterial moiety having a long-lasting antibacterial effect, the aqueous solution having sufficient stability over time.

### Background Art

In recent years, people have been paying more attention to cleanliness in response to improvement in living environments and increased health consciousness. Even in everyday lives, people actively take various measures to prevent infection of humans and animals with viruses and pathogenic bacteria. People are especially sensitive to the presence of influenza viruses, which have a great impact on not only the infected individuals, but also family life, schools, businesses, and even society as a whole, bacteria such as pathogenic E. Coli bacteria (such as O-157) that may cause serious symptoms in the infected individuals and methicillin resistant Staphylococcus aureus (MRSA), and fungi such as eumycetes (molds) that are common in everyday lives. For example, the eumycetes are commonly grown on, for example, foods and textiles such as clothes and carpets in our everyday lives and may cause symptoms such as atopic dermatitis. In this context, there has been an increasing demand for daily-use household items that are previously rendered antibacterial and antiviral and products that can readily inhibit the growth of bacteria or destroy bacteria on daily-use household items. To meet the demand, many antibacterial and antiviral agents that can readily provide antibacterial and antiviral treatment to a target object have been commercially available.

However, conventional antibacterial and antiviral agents exhibit a short duration of the antibacterial and antiviral effect. And it has been noted that the conventional agents have a disadvantage in that their antibacterial effect does not last long enough. For example, the conventional agents volatilize, degrade, or dissolved over time, which results in gradual loss of their antibacterial effectiveness.

In this regard, in recent years, antibacterial agents that use a silane compound (hereinafter also referred to as silicon-containing compound) that has an antibacterial moiety (antibacterial functional group) have been proposed, in place of the conventional antibacterial and antiviral agents (see, for example, Patent document 1).

Specifically, Patent document 1 discloses an antibacterial agent that uses a silane compound that has a quaternary ammonium salt as an antibacterial functional group. Patent document 1 also discloses a technique for immobilizing the antibacterial agent on the surface of a target object to be rendered antibacterial (hereinafter referred to as target surface) by diluting the antibacterial agent with an alcohol or the like to prepare an antibacterial agent solution and immersing the target object in the solution. Use of such technique for immobilizing the antibacterial agent on a target surface can provide a long-lasting antibacterial effect, because the silane compound is immobilized on the target surface by covalent attachment.

The antibacterial agent can also be immobilized on a target surface by dissolving the silane compound that has the antibacterial functional group in a predetermined solvent to prepare an antibacterial agent solution and applying the antibacterial agent solution onto a predetermined target surface. Conventionally, formulations that include a silane compound that has the antibacterial functional group have been commercially available. It is necessary that the formulations include a high level of alcohol for the component properties, the functionality, and the stability of the formulations. However, use of an alcohol, which is flammable, in the production process requires specialized equipment such as explosion-proof equipment and has limitations in storage, maintenance, the amount of the material, and transportation, which incurs extra costs. Applications of silane compounds that have an antibacterial functional group include, for example, disinfection of hands and oral care such as breath freshening and cavity protection. As there is an increasing need for direct application to human bodies (including animal bodies), the compounds that include an alcohol may be avoided.

Patent document 2 describes a technique for diluting, with water rather than an organic solvent, a raw solution (an alcohol solution) that includes a silane compound that has an antibacterial functional group to consider the environmental load.

Specifically, Patent document 2 describes a stable, aqueous antibacterial solution that exhibits no viscosity change, the solution prepared by diluting a raw solution of an antibacterial agent with water to prepare an aqueous solution of the antibacterial agent at a predetermined concentration, adding an acid such as hydrochloric acid to the aqueous solution to adjust the pH to a range of from 3.5 to 2.0, and storing the aqueous solution at a temperature equal to or lower than 10°C.

### Citation List

### Patent Literature

PTL 1: Japanese Unexamined Patent Application Publication No. 2004-209241
PTL 2: Japanese Unexamined Patent Application Publication No. 2007-31290

### Summary of Invention

### Technical Problem

However, the aqueous solution of the antibacterial agent described in Patent document 2 requires pH adjustment and maintenance at low temperature to inhibit a change of the aqueous solution over time. Thus, even if the antibacterial agent is dissolved to prepare an aqueous solution at a predetermined concentration, it is difficult to stably store the aqueous solution and to dissolve the antibacterial agent in water without losing the stability over time. Thus, there is a desire for antibacterial and antiviral agents that require no special conditions to store the agents, that can be applied to an object where use of an organic solvent such as an alcohol is avoided, and that include an aqueous solution that has high stability over time.

Therefore, it is an object of the present invention to provide an aqueous solution of a silicon-containing compound that has an antibacterial functional group, the aqueous solution having high stability over time, and an antiviral and antibacterial agent that includes the aqueous solution.

### Solution to Problem

The problems to be solved by the present invention have been described above. Now, means of solving the problems will be described.

An aqueous solution of a silicon-containing compound according to the present invention includes a silicon-containing compound and a cyclodextrin (hereinafter sometimes abbreviated as CD) or a cyclodextrin derivative.

In the aqueous solution of a silicon-containing compound according to the present invention, the silicon-containing compound is represented by the following general formula (1): 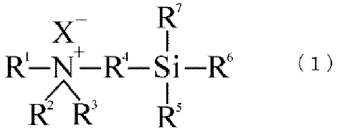 (in which R1 represents a hydrocarbon group having 6 or more carbon atoms, R2 and R3 are optionally the same or different and represent a lower hydrocarbon group, R4 represents a divalent lower hydrocarbon group, R5, R6, and R7 are optionally the same or different and represent a lower alkyl group or a lower alkoxy group, and X represents a halogen ion or an organic carbonyloxy ion).

The aqueous solution of a silicon-containing compound according to the present invention includes the silicon-containing compound in an amount equal to or more than at least 0.001 % by weight.

The aqueous solution of a silicon-containing compound according to the present invention includes the silicon-containing compound and the cyclodextrin or the cyclodextrin derivative at a weight ratio of from 0.1:30 to 10:1.

In the aqueous solution of a silicon-containing compound according to the present invention, the cyclodextrin or the cyclodextrin derivative is at least one selected from the group consisting of α-cyclodextrin, β-cyclodextrin, γ-cyclodextrin, branched cyclodextrins, and complexed cyclodextrins.

In the aqueous solution of a silicon-containing compound according to the present invention, the silicon-containing compound represented by the general formula (1) may include octadecyl dimethyl(3-triethoxysilylpropyl)ammonium chloride.

An antibacterial and antiviral agent according to the present invention includes the aqueous solution of a silicon-containing compound as described above.

### Advantageous Effects of Invention

According to the present invention, inclusion of a silicon-containing compound itself as a guest molecule in a cyclodextrin or a cyclodextrin derivative allows dissolution of the silicon-containing compound and provision of an antibacterial and antiviral agent that includes an aqueous solution that has high stability over time. As a result, an object where use of an organic solvent such as an alcohol is avoided can be rendered antibacterial and antiviral.

### Brief Description of Drawings

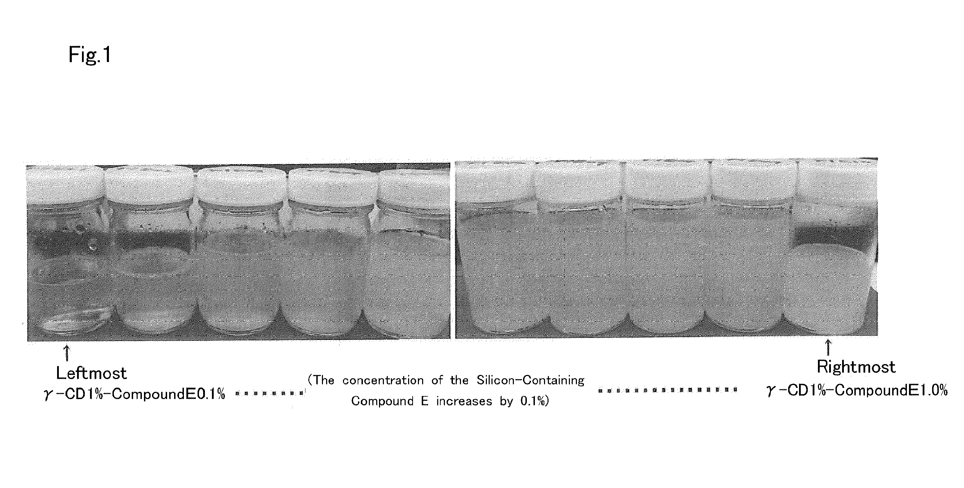

Fig.1 is a photograph illustrating aqueous solutions of γ-CD and Silicon-Containing Compound E (ratio of CD alone: 1% by weight, ratio of Silicon-Containing Compound E: 0.1 to 1.0% by weight).

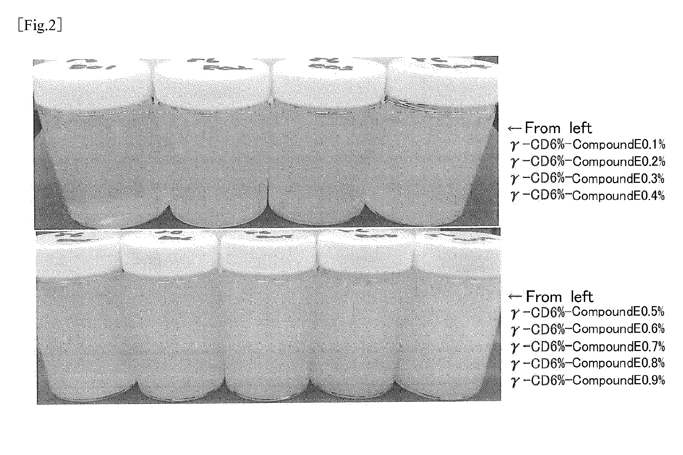

Fig.2 is a photograph illustrating aqueous solutions of γ-CD and Silicon-Containing Compound E (ratio of CD alone: 6% by weight, ratio of Silicon-Containing Compound E: 0.1 to 0.9% by weight).

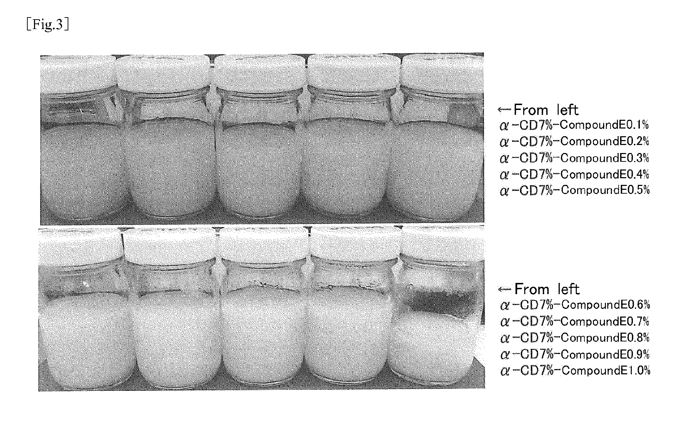

Fig.3 is a photograph illustrating aqueous solutions of γ-CD and Silicon-Containing Compound E (ratio of CD alone: 7% by weight, ratio of Silicon-Containing Compound E: 0.1 to 1.0% by weight).

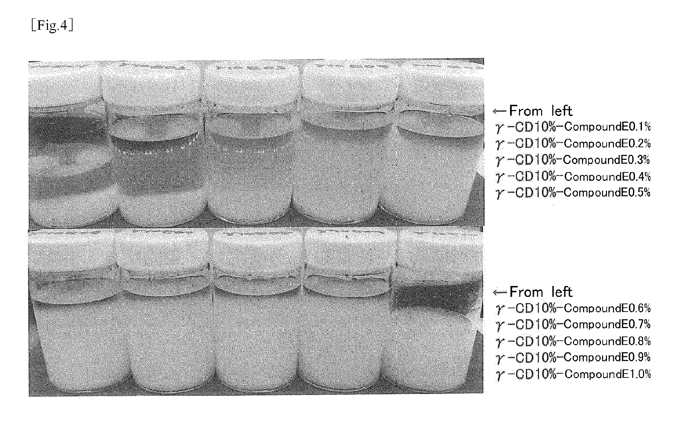

Fig.4 is a photograph illustrating aqueous solutions of γ-CD and Silicon-Containing Compound E (ratio of CD alone: 10% by weight, ratio of Silicon-Containing Compound E: 0.1 to 1.0% by weight).

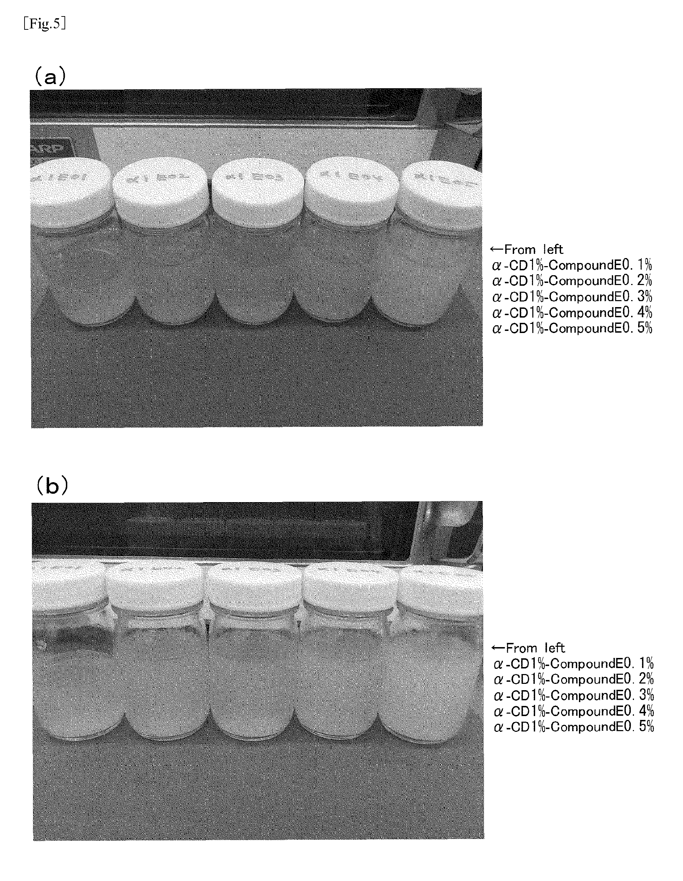

Figs.5(a) and 5(b) are a photograph illustrating aqueous solutions of α-CD and Silicon-Containing Compound E (ratio of CD alone: 1% by weight, ratio of Silicon-Containing Compound E: 0.1 to 0.5% by weight). Fig.5(a) is a photograph taken from a perspective above, and Fig.5(b) is a photograph taken from the front.

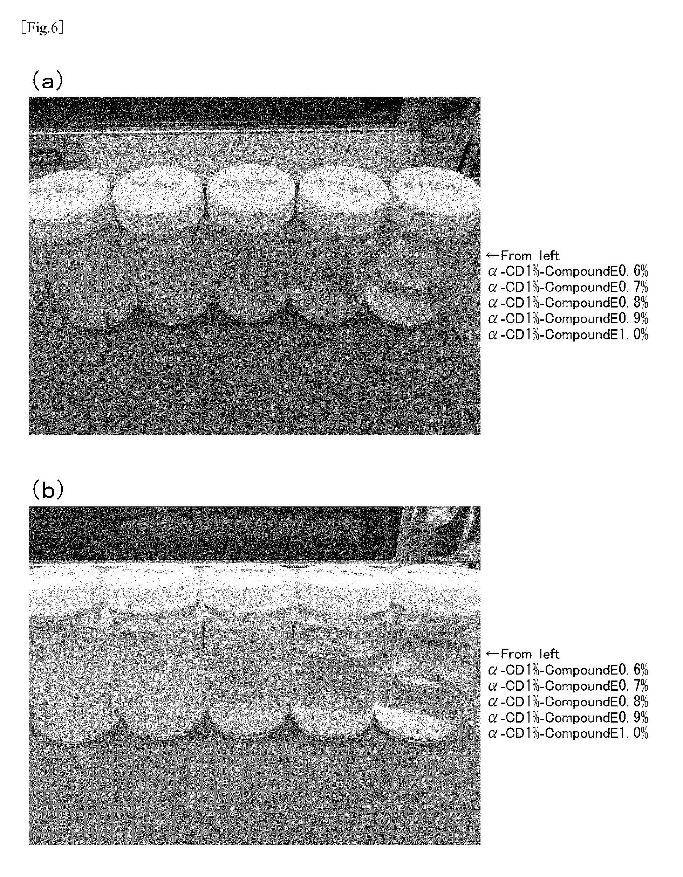

Figs.6(a) and 6(b) are a photograph illustrating aqueous solutions of α-CD and Silicon-Containing Compound E (ratio of CD alone: 1% by weight, ratio of Silicon-Containing Compound E: 0.6 to 1.0% by weight). Fig.6(a) is a photograph taken from a perspective above, and Fig.6(b) is a photograph taken from the front.

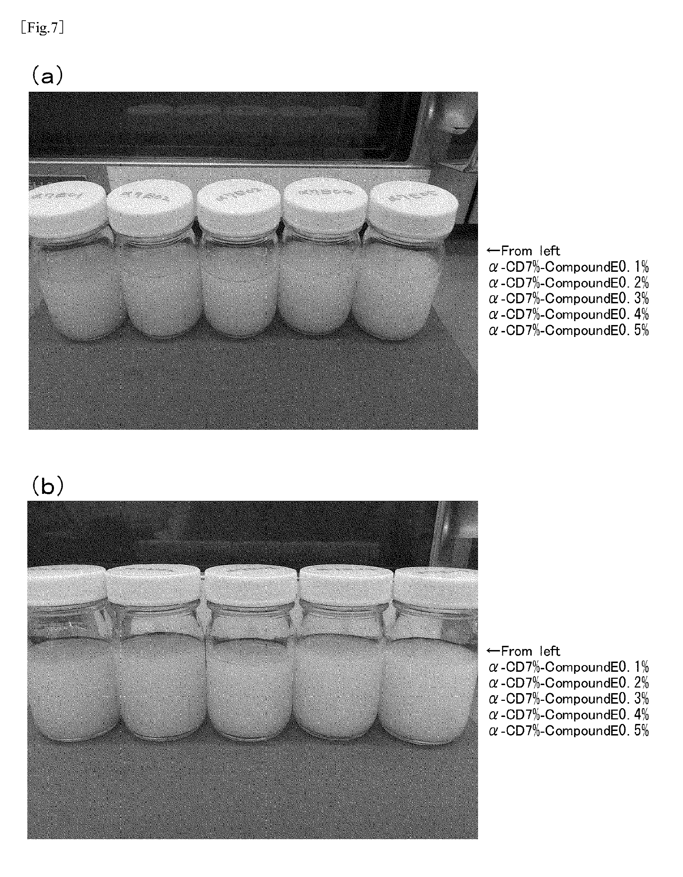

Figs.7(a) and 7(b) are a photograph illustrating aqueous solutions of α-CD and Silicon-Containing Compound E (ratio of CD alone: 7% by weight, ratio of Silicon-Containing Compound E: 0.1 to 0.5% by weight). Fig.7(a) is a photograph taken from a perspective above, and Fig.7(b) is a photograph taken from the front.

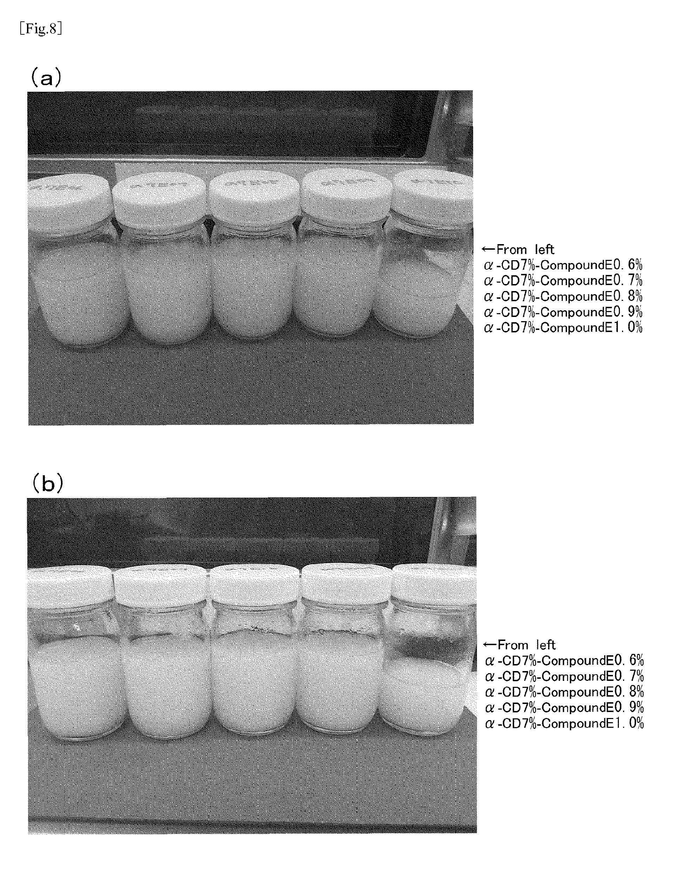

Figs.8(a) and 8(b) are a photograph illustrating aqueous solutions of α-CD and Silicon-Containing Compound E (ratio of CD alone: 7% by weight, ratio of Silicon-Containing Compound E: 0.6 to 1.0% by weight). Fig.8(a) is a photograph taken from a perspective above, and Fig.8(b) is a photograph taken from the front.

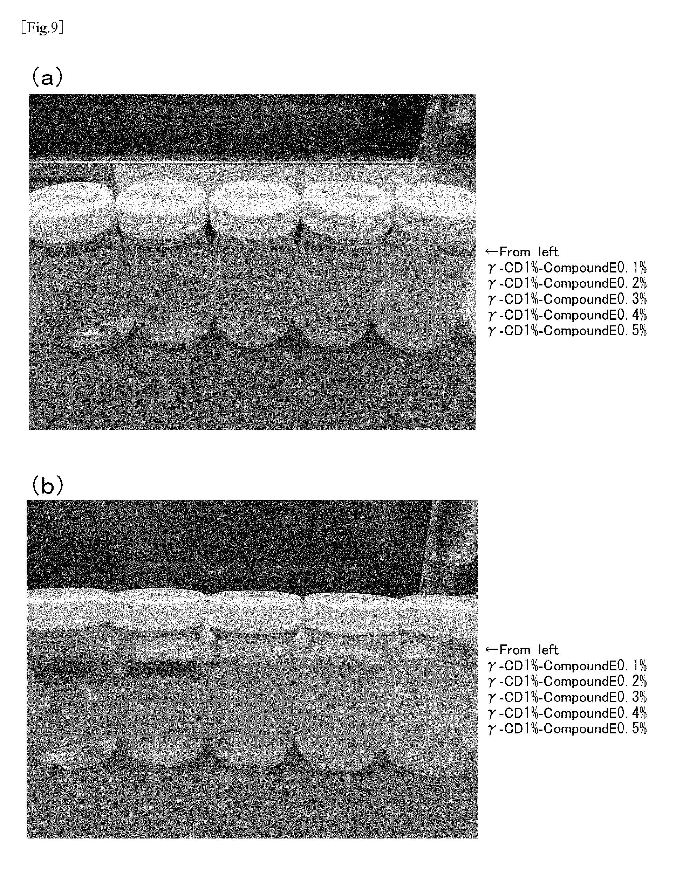

Figs.9(a) and 9(b) are a photograph illustrating aqueous solutions of γ-CD and Silicon-Containing Compound E (ratio of CD alone: 1% by weight, ratio of Silicon-Containing Compound E: 0.1 to 0.5% by weight). Fig.9(a) is a photograph taken from a perspective above, and Fig.9(b) is a photograph taken from the front.

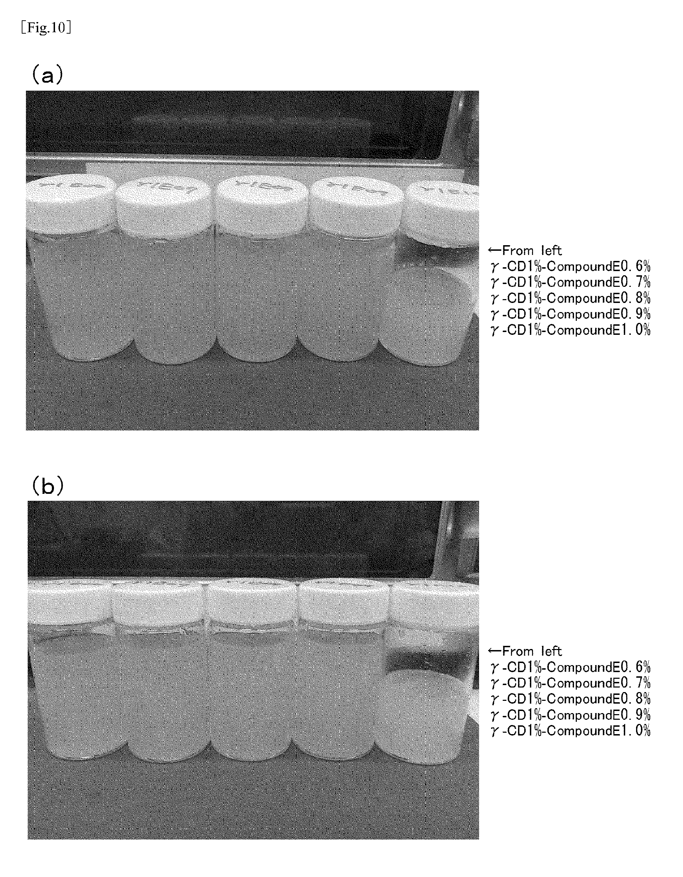

Figs.10(a) and 10(b) are a photograph illustrating aqueous solutions of γ-CD and Silicon-Containing Compound E (ratio of CD alone: 1% by weight, ratio of Silicon-Containing Compound E: 0.6 to 1.0% by weight). Fig.10(a) is a photograph taken from a perspective above, and Fig.10(b) is a photograph taken from the front.

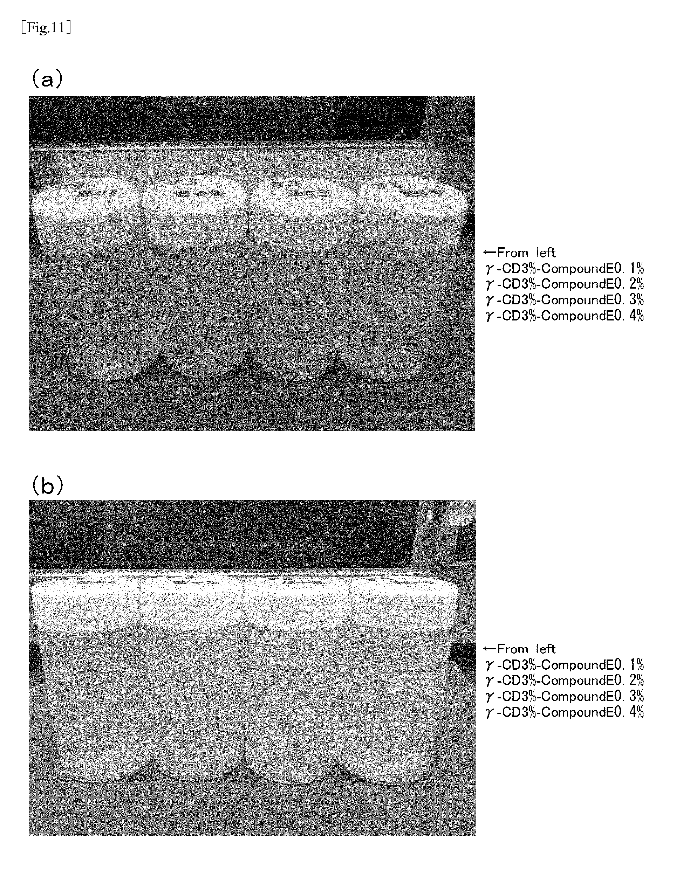

Figs.11(a) and 11(b) are a photograph illustrating aqueous solutions of γ-CD and Silicon-Containing Compound E (ratio of CD alone: 3% by weight, ratio of Silicon-Containing Compound E: 0.1 to 0.4% by weight). Fig.11(a) is a photograph taken from a perspective above, and Fig.11(b) is a photograph taken from the front.

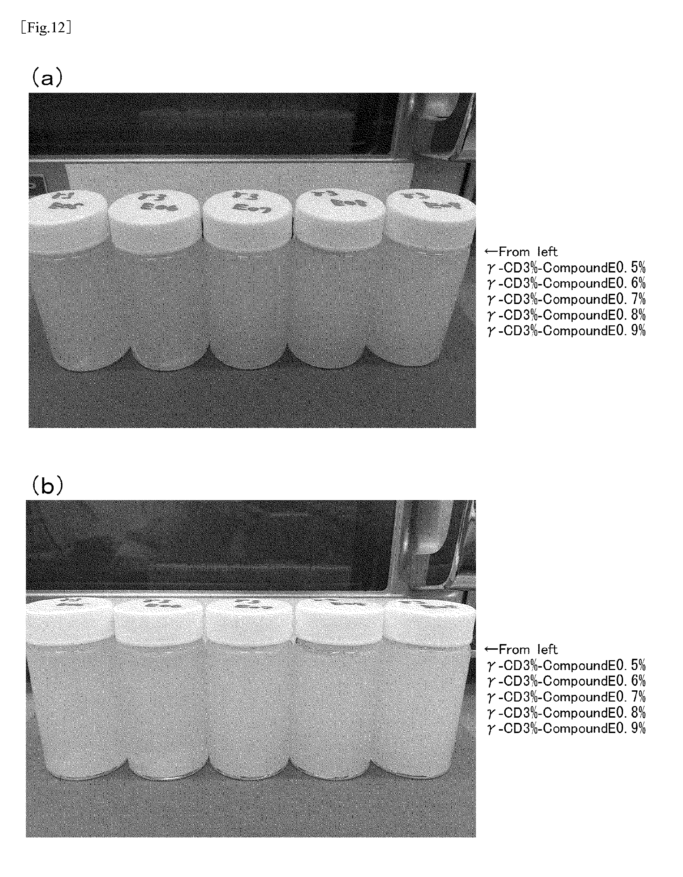

Figs.12(a) and 12(b) are a photograph illustrating aqueous solutions of γ-CD and Silicon-Containing Compound E (ratio of CD alone: 3% by weight, ratio of Silicon-Containing Compound E: 0.5 to 0.9% by weight). Fig.12(a) is a photograph taken from a perspective above, and Fig.12(b) is a photograph taken from the front.

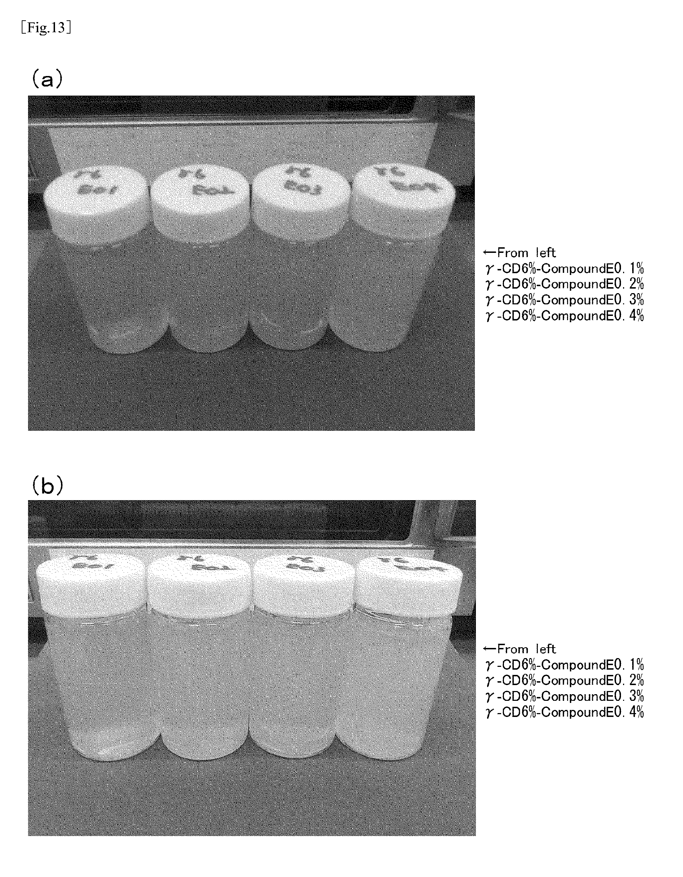

Figs.13(a) and 13(b) are a photograph illustrating aqueous solutions of γ-CD and Silicon-Containing Compound E (ratio of CD alone: 6% by weight, ratio of Silicon-Containing Compound E: 0.1 to 0.4% by weight). Fig.13(a) is a photograph taken from a perspective above, and Fig.13(b) is a photograph taken from the front.

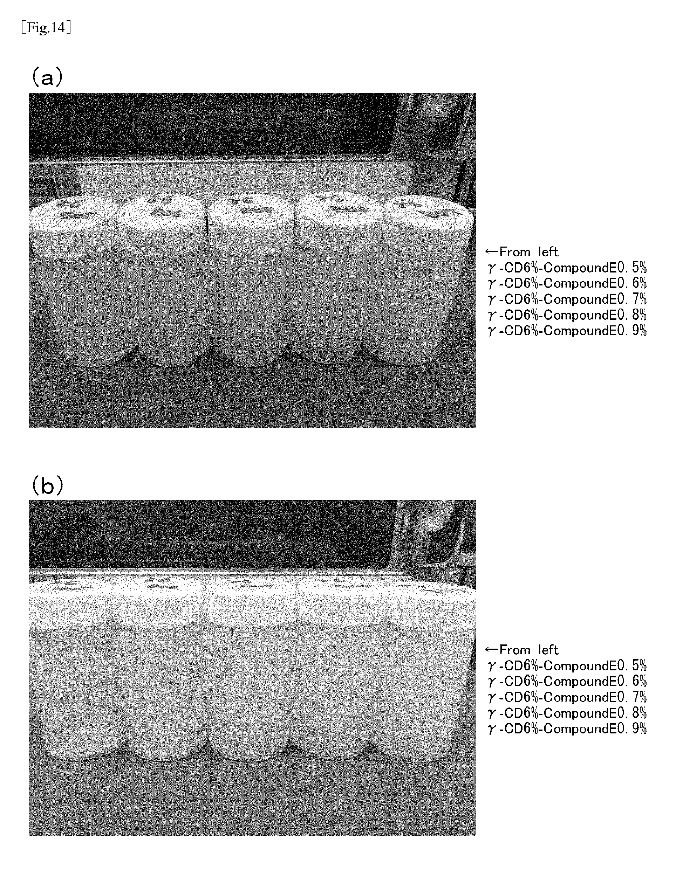

Figs.14(a) and 14(b) are a photograph illustrating aqueous solutions of γ-CD and Silicon-Containing Compound E (ratio of CD alone: 6% by weight, ratio of Silicon-Containing Compound E: 0.5 to 0.9% by weight). Fig.14(a) is a photograph taken from a perspective above, and Fig.14(b) is a photograph taken from the front.

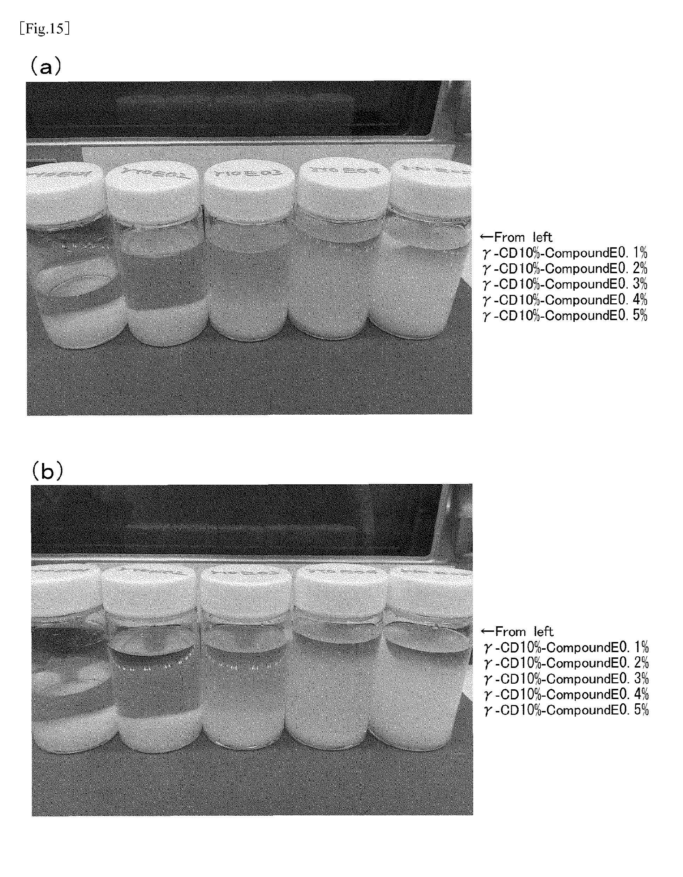

Figs.15(a) and 15(b) are a photograph illustrating aqueous solutions of γ-CD and Silicon-Containing Compound E (ratio of CD alone: 10% by weight, ratio of Silicon-Containing Compound E: 0.1 to 0.5% by weight). Fig.15(a) is a photograph taken from a perspective above, and Fig.15(b) is a photograph taken from the front.

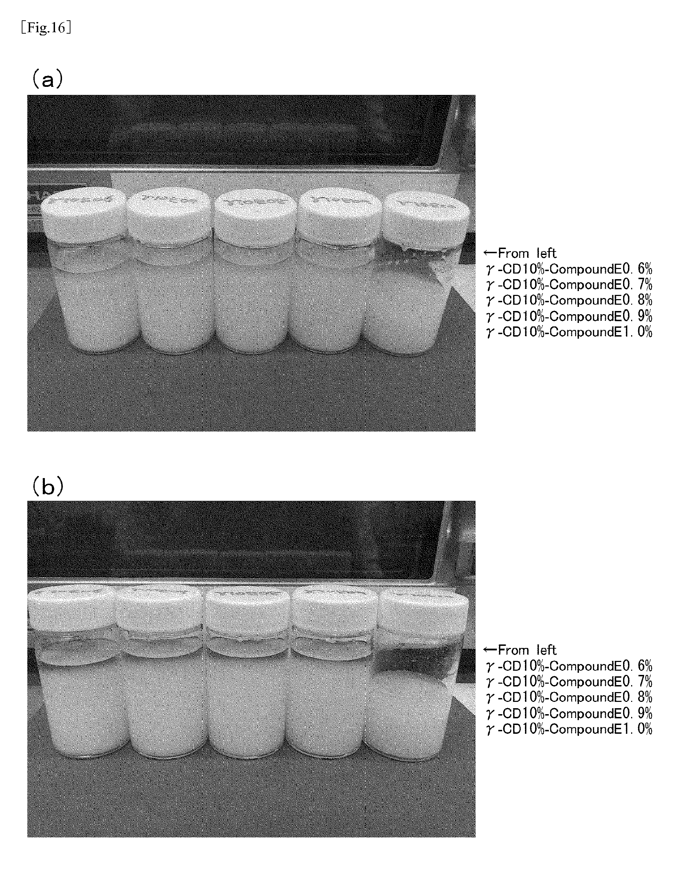

Figs.16(a) and 16(b) are a photograph illustrating aqueous solutions of γ-CD and Silicon-Containing Compound E (ratio of CD alone: 10% by weight, ratio of Silicon-Containing Compound E: 0.6 to 1.0% by weight). Fig.16(a) is a photograph taken from a perspective above, and Fig.16(b) is a photograph taken from the front.

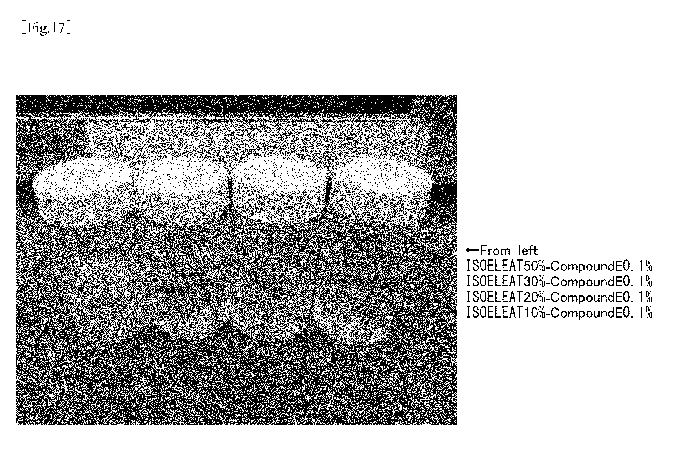

Fig.17 is a photograph illustrating aqueous solutions of ISOELEAT and Silicon-Containing Compound E (ratio of CD alone: 10 to 50% by weight, ratio of Silicon-Containing Compound E: 0.1% by weight). Fig.17(a) is a photograph taken from a perspective above, and Fig.17(b) is a photograph taken from the front.

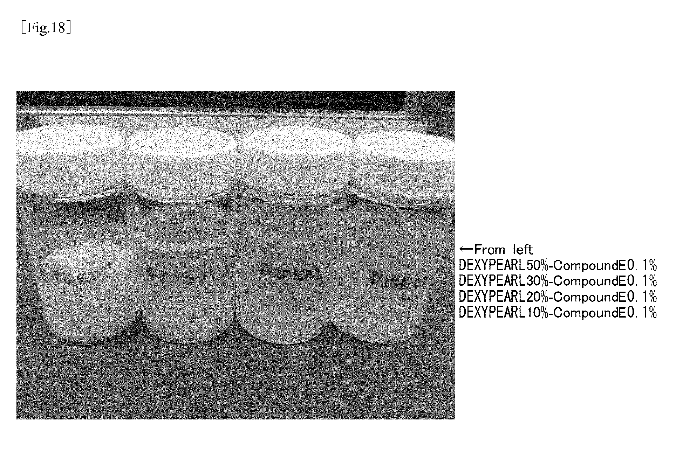

Fig.18 is a photograph illustrating aqueous solutions of DEXYPEARL and Silicon-Containing Compound E (ratio of CD alone: 10 to 50% by weight, ratio of Silicon-Containing Compound E: 0.1% by weight). Fig.18(a) is a photograph taken from a perspective above, and Fig.18(b) is a photograph taken from the front.

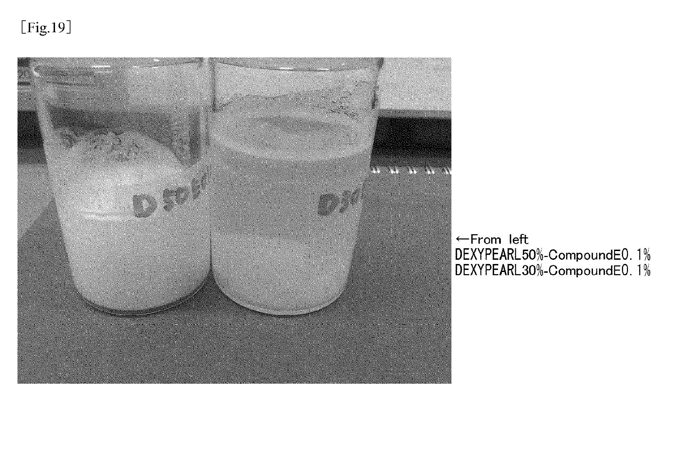

Fig.19 is a photograph comparing precipitation in the aqueous solution of 50% DEXYPEARL and 0.1% Compound E and precipitation in the aqueous solution of 30% DEXYPEARL and 0.1 % Compound E illustrated in Fig.18.

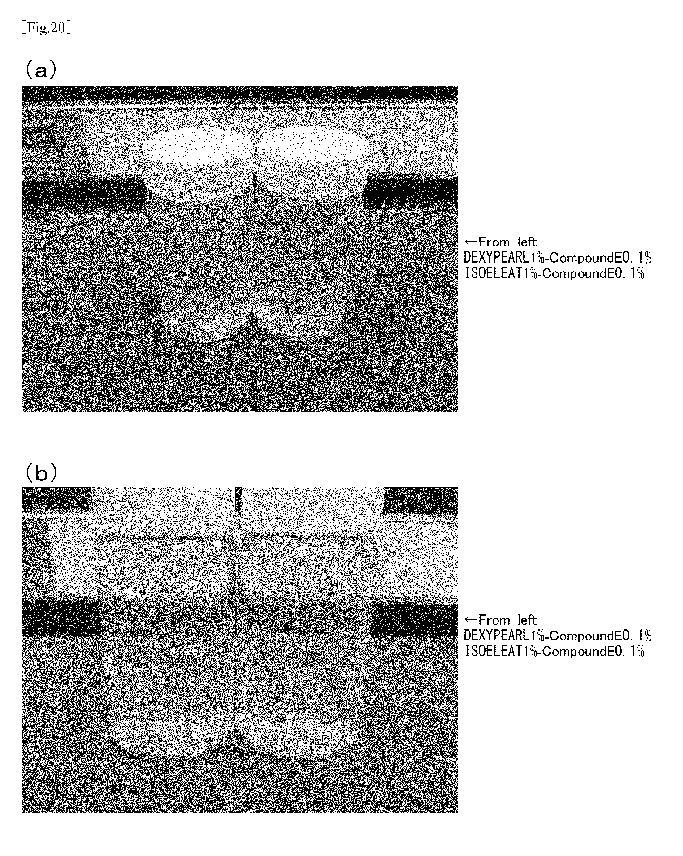

Figs.20(a) and 20(b) are a photograph comparing the aqueous solution of 1% DEXYPEARL and 0.1 % Compound E and the aqueous solution of 1% ISOELEAT and 0.1 % Compound E. Fig.20(a) is a photograph taken from a perspective above, and Fig.20(b) is a photograph taken from the front.

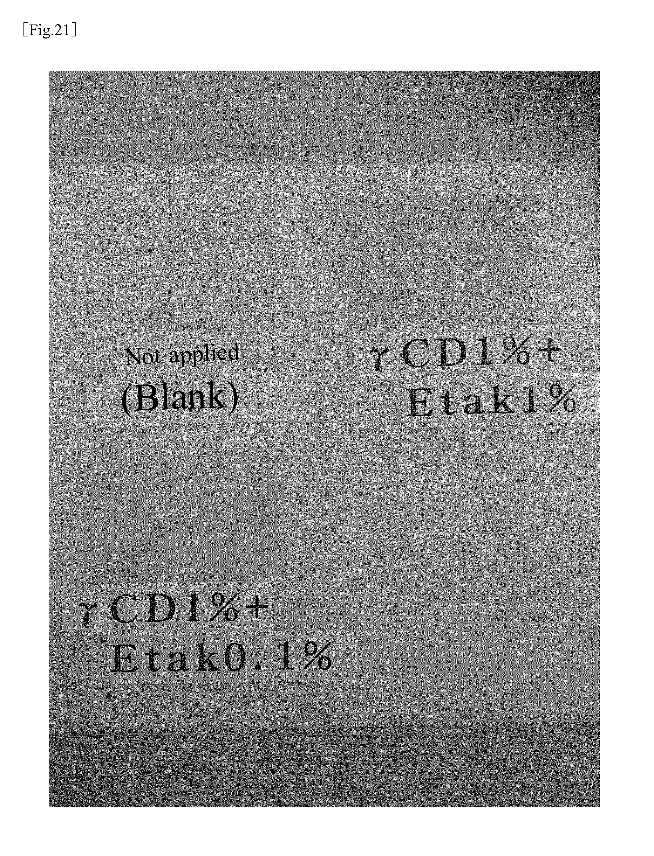

Fig.21 is a photograph illustrating the results of qualitative tests using a bromophenol blue solution.

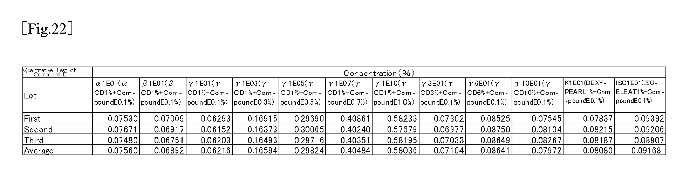

Fig.22 is a table illustrating the results of quantitative analysis of Silicon-Containing Compound E in aqueous solutions of Silicon-Containing Compound E in stability tests (after 3 months).

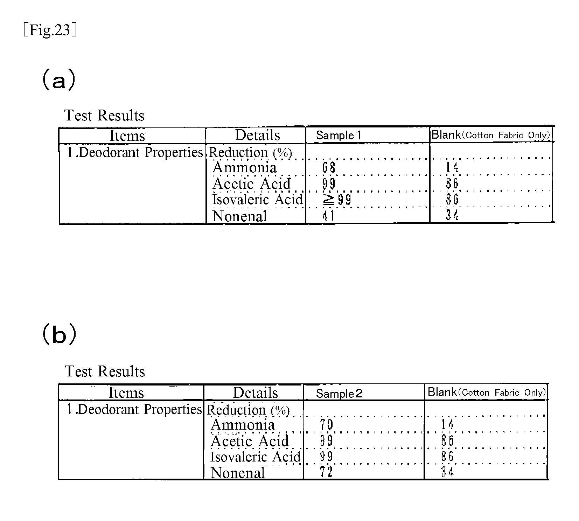

Figs.23(a) and 23(b) are a table illustrating the results of deodorant activity tests. Fig.23(a) is a table illustrating the results of a sample 1, and Fig.23(b) is a table illustrating the results of a sample 2.

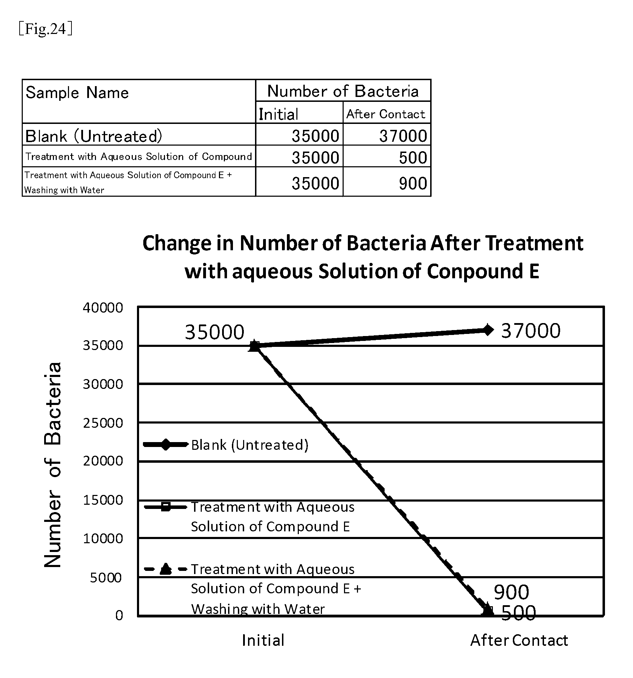

Fig.24 is a graph illustrating the results of antibacterial evaluation tests.

### Description of Embodiments

Next, embodiments of the present invention will be described.

In the present invention, "antibacterial" is to be taken in its broadest context, including destruction and removal of bacteria and eumycetes and inhibition of development, growth, and proliferation of bacteria and eumycetes, and is not limited in any way. And "antiviral" is to be taken in its broadest context, including prevention of infection with pathogenic viruses, inactivation of pathogenic viruses, and inhibition of proliferation of pathogenic viruses, and is not limited in any way.

An aqueous solution of a silicon-containing compound according to the present invention includes a silicon-containing compound and a cyclodextrin or a cyclodextrin derivative.

The silicon-containing compound is represented by the following general formula (1): 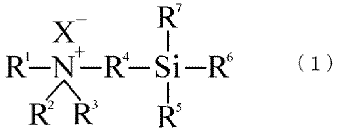 (in which, R1 represents a hydrocarbon group having 6 or more carbon atoms, R2 and R3 are optionally the same or different and represent a lower hydrocarbon group, R4 represents a divalent lower hydrocarbon group, R5, R6, and R7 are optionally the same or different and a lower alkyl group or a lower alkoxy group, and X represents a halogen ion or an organic carbonyloxy ion).

Next, with regard to the aqueous solution of a silicon-containing compound and an antibacterial and antiviral agent that includes the aqueous solution according to the present invention, the silicon-containing compound, which is an active ingredient having an antibacterial and antiviral effect, and the cyclodextrin or the cyclodextrin derivative, which makes the silicon-containing compound water-soluble will be individually described.

The silicon-containing compound is represented by the general formula (1) and has an antibacterial moiety (antibacterial functional group).

As represented by the general formula (1), the silicon-containing compound that has an antibacterial moiety (antibacterial functional group) is an organic silane compound that includes a quaternary ammonium salt, which is the antibacterial functional group (antibacterial active moiety), the salt being linked via a lower alkylene group to a silane compound that can form a covalent bond with oxygen. Thus, the silicon-containing compound according to the present invention includes, in its molecule, a quaternary ammonium salt, which is known as an antibacterial ingredient. Due to the quaternary ammonium salt, the compound has a strong antibacterial and antiviral effect. The silicon-containing compound according to the present invention also includes, in its molecule, a silane compound that can form a covalent bond with oxygen. The ability for the silane compound to form a covalent bond with oxygen on a target surface allows firm immobilization of the quaternary ammonium salt, which is an antibacterial ingredient, on the target surface, thereby providing a strong antibacterial effect to the target surface and a longer duration of the effect.

In preferred embodiments of the silicon-containing compound represented by the general formula (1), R1 in the general formula (1) represents an alkyl group having 10 to 25 carbon atoms, R2 and R3 are optionally the same or different and represent a lower alkyl group having 1 to 6 carbon atoms, R4 represents a lower alkylene group having 1 to 6 carbon atoms, R5, R6, and R7 are optionally the same or different and represent a lower alkyl group or a lower alkoxy group having 1 to 6 carbon atoms, and X is a halogen ion or an organic carbonyloxy ion (organic carboxylate ion).

Examples of the hydrocarbon group having 6 or more carbon atoms as R1 can include a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, an uneicosyl group, a doeicosyl group, a trieicosyl group, a tetraeicosyl group, and a pentaeicosyl group.

Examples of the lower hydrocarbon group as R2 and R3, which are optionally the same or different, can include, for example, a methyl group, an ethyl group, a propyl group, an isopropyl group, a butyl group, a pentyl group, a hexyl group, a cyclohexyl group, a phenyl group, and a tolyl group.

Examples of the lower alkylene group as R4 can include a methylene group, an ethylene group, a trimethylene group, a tetramethylene group, and a hexamethylene group.

R5, R6, and R7 are optionally the same or different and a lower alkyl group or a lower alkoxy group. The specific examples can include a methoxy group, an ethoxy group, a propoxy group, an isopropoxy group, a butoxy group, a pentyloxy group, a hexyloxy group, a methyl group, an ethyl group, a propyl group, an isopropyl group, a butyl group, a pentyl group, and a hexyl group.

Examples of X can include halogen ions such as a chlorine ion and a bromine ion, and organic carbonyloxy ions (organic carboxylate ions) such as a methylcarbonyloxy ion (acetate ion), an ethylcarbonyloxy ion (propionate ion), and a phenylcarbonyloxy ion (benzoate ion).

Specific examples of the silicon-containing compound can include the silicon-containing compounds (a) represented by the general formula (1), the compounds selected from the group consisting of octadecyl dimethyl(3-trimethoxysilylpropyl)ammonium chloride, octadecyl dimethyl(3-triethoxysilylpropyl)ammonium chloride, octadecyl diethyl(3-trimethoxysilylpropyl) ammonium chloride, octadecyl dimethyl(2-trimethylsilylethyl)ammonium chloride, octadecyl dipropyl(4-trimethoxysilylbutyl)ammonium acetate, octadecyl dimethyl(3-triisopropoxysilylpropyl)ammonium chloride, octadecyl dimethyl(3-triethylsilylpropyl)ammonium chloride, octadecyl dimethyl(3-triisopropylsilylpropyl)ammonium chloride, heptadecyl dimethyl(3-trimethoxysilylpropyl)ammonium chloride, heptadecyl diisopropyl (2-triethoxysilylethyl)ammonium chloride, hexadecyl dimethyl(3-trimethoxysilylpropyl)ammonium chloride, hexadecyl dimethyl(3-trimethoxysilylpropyl)ammonium acetate, and pentadecyl dimethyl(3-triethoxysilylpropyl)ammonium chloride. Among them, octadecyl dimethyl(3-triethoxysilylpropyl)ammonium chloride (also known as octadecylamino dimethyl triethoxysilylpropyl ammonium chloride) is preferred.

In the present invention, at least one of the above silicon-containing compounds is used, while two or more of the silicon-containing compounds may be used in combination, if appropriate.

As the silicon-containing compound, octadecyl dimethyl(3-triethoxysilylpropyl)ammonium chloride is especially preferred.

The silicon-containing compounds described above can be prepared according to any know method without limitation.

Next, the cyclodextrin or the cyclodextrin derivative used in the present invention will be described.

The cyclodextrin or the cyclodextrin derivative used in the present invention can include the silicon-containing compound into a space within the molecule of the cyclodextrin or the cyclodextrin derivative. And the cyclodextrin or the cyclodextrin derivative has deodorant properties due to the effect of trapping an odor-causing substance (molecule).

Preferred examples of the cyclodextrin include, but not limited to, α-cyclodextrin, β-cyclodextrin, and γ-cyclodextrin. Examples of the cyclodextrin derivative include, for example, branched cyclodextrins produced by introducing a substituent such as an alkyl group, an acetyl group, a trityl group, a tosyl group, a trimethylsilane group, a phenyl group, and a halogen group to add a branch to the cyclodextrin ring (for example, ISOELEAT P commercially available from Ensuiko Sugar Refining Co., Ltd.), and complexed cyclodextrins that are a cyclodextrin mixture that includes α-cyclodextrin as a major component (for example, DEXYPEARL K-100 commercially available from Ensuiko Sugar Refining Co., Ltd.). Desirably, the cyclodextrin or the cyclodextrin derivative according to the present invention is at least one selected from the group consisting of the above cyclodextrins or the above cyclodextrin derivatives.

Next, the antibacterial and antiviral agent, which is an aqueous solution that includes the silicon-containing compound and the cyclodextrin or the cyclodextrin derivative, will be described.

The antibacterial and antiviral agent according to the present invention exerts an antibacterial and antiviral effect against, for example, deleterious bacteria such as E. Coli O-157, MRSA (methicillin resistant Staphylococcus aureus), and legionella, eumycetes (molds) that cause mycoses and mycotoxicoses, and algae, through the silicon-containing compound included in the antibacterial and antiviral agent.

The antibacterial and antiviral agent according to the present invention exerts a deodorant effect (deodorant activity) against, for example, an odor source, through the cyclodextrin or the cyclodextrin derivative included in the antibacterial and antiviral agent.

The deodorant effect (deodorant activity) of the antibacterial and antiviral agent according to the present invention refers to the ability to adsorb odor, namely, the ability to act on an odor source such as, for example, a volatile hydrophobic organic compound to trap the odor source such as a volatile hydrophobic organic compound in a space within the molecule, and the effect is not to be construed as limiting in any way.

The objects to be treated include any objects that are desired to be rendered antibacterial and antiviral and to be deodorized (target objects), including, for example, various industrial products made of, for example, wood, glass, fibers, metal, ceramics, and rubber, daily-use items such as cosmetics and toiletry items, human skin such as hands, and animals such as pets.

Examples of a method for rendering a target object antibacterial and antiviral and deodorizing a target object include spraying, application by a sheet applicator such as a wet wipe, dropwise application by a pump bottle, brushing, rolling, and dip coating (application by immersion).

The antibacterial and antiviral agent according to the present invention may include one of the silicon-containing compounds, which are an active ingredient, or may include two or more of the compounds in combination, if appropriate. The antibacterial and antiviral agent may also include another additive. The antibacterial and antiviral agent may take any form as long as the agent is an aqueous solution that includes the silicon-containing compound and the cyclodextrin or the cyclodextrin derivative. Depending on the form of the agent, the additive may be one or more selected from the group consisting of moisturizers, emulsifiers, dispersing agents, antioxidants, ultraviolet light absorbers, fragrances, and the like, to the extent that the additive does not inhibit the effects of the present invention.

Examples of the emulsifiers and the dispersing agents that can be used include common surfactants, including, for example, anionic surfactants such as fatty acid sodium, sodium alpha sulfo fatty acid esters, sodium linear alkyl benzene sulfonates, sodium alkyl sulfates, sodium alkyl ether sulfates, sodium alpha olefin sulfonates, and sodium alkyl sulfonates, nonionic surfactants such as sucrose fatty acid esters, sorbitan fatty acid esters, polyoxyethylene sorbitan fatty acid esters, fatty acid alkanolamides, polyoxyethylene alkyl ethers, polyoxyethylene alkyl phenyl ethers, polyoxyethylene alkyl esters, polyoxyethylene sorbitan alkyl esters, and sorbitan alkyl esters, amphoteric surfactants such as sodium alkyl amino fatty acids, alkyl betaines, and alkyl amine oxides, and cationic surfactants such as alkyl trimethyl ammonium salts and dialkyl dimethyl ammonium salts.

Examples of the antioxidants include tocopherol (vitamin E), tocopherol derivatives such as tocopherol acetate, phenol-based antioxidants (such as 2,6-di-t-butyl-p-cresol and 2,2'-methylene bis(4-methyl-6-t-butylphenol)), sulfur-based antioxidants (such as dilauryl 3,3'-thiodipropionate and distearyl 3,3'-thiodipropionate), phosphorous-based antioxidants (such as triphenyl phosphite and triisodecyl phosphite), and amine-based antioxidants (such as octylated diphenyl amine, N-n-butyl-p-aminophenol, and N,N-diisopropyl-p-phenylene diamine).

Examples of the ultraviolet light absorbers include benzophenone-based absorbers (such as 2-hydroxybenzophenone and 2,4-dihydroxybenzophenone) and benzotriazole-based absorbers (such as (2'-hydroxyphenyl)benzotriazole and (2'-hydroxy-5'-methylphenyl)benzotriazole).

An example of the fragrances that can be used includes tea tree oil, which is an essential oil derived from Metrosideros (Myrtacea), Psidium, Eucalyptus, Leptospermum, or Cajeput plants. And one or more of the tea tree oil, anise oil, orange oil, clove oil, citronella oil, jasmine oil, camphor oil, spearmint oil, geranium oil, turpentine oil, sandalwood oil, peppermint oil, bergamot oil, rosewood oil, eucalyptus oil, lavender oil, lemongrass oil, lemon oil, rose oil, musk, civet, castor, ambergris, anethole, eugenol, geraniol, citronellol, and mentha oil can be added.

The amount of the silicon-containing compound to be added and the method for adding the compound may vary depending on, for example, the type, the material, and the intended-use of a target object to be rendered antibacterial and antiviral. In general, the silicon-containing compound is included in an amount equal to or more than at least 0.001% by weight, preferably from 0.01 to 1.0% by weight, and more preferably from 0.05 to 0.1% by weight, based on the total composition of the product.

In preparation of the aqueous solution of the silicon-containing compound, the silicon-containing compound and the cyclodextrin or the cyclodextrin derivative may be included at any weight ratio, while preferably, the silicon-containing compound and the cyclodextrin or the cyclodextrin derivative are included at a weight ratio of from 0.1:30 to 10:1 and more preferably from 0.1:20 to 6:1.0.

The amount of the cyclodextrin or the cyclodextrin derivative to be added and the method for adding the cyclodextrin or the cyclodextrin derivative may vary depending on, for example, the type, the material, and the intended-use of a target object to be rendered antibacterial and antiviral. The ratio of the cyclodextrin or the cyclodextrin derivative is dictated by the water solubility of the cyclodextrin or the cyclodextrin derivative. Specifically, for example, α-cyclodextrin, β-cyclodextrin, γ-cyclodextrin, complexed cyclodextrins, and branched cyclodextrins are respectively included preferably in an amount of from 1 to 7% by weight, from 0.1 to 1% by weight, from 1 to 10% by weight, from 1 to 20% by weight, and from 1 to 9% by weight, based on the total composition of the product.

As β-cyclodextrin is difficult to dissolve in water, β-cyclodextrin is included in an amount equal to or less than 1% by weight, as described above.

Examples of applications of the aqueous solution of the silicon-containing compound according to the present invention and the antibacterial and antiviral agent that includes the aqueous solution include, for example, disinfection of hands and various oral care such as breath freshening and cavity protection. In addition to direct application to human bodies (including animal bodies), the solution and the agent can be applied to the surface of objects that are desired to be rendered antibacterial and antiviral. Examples of the objects can include a wide range of articles including dental articles such as implants, crowns, bridges, orthodontic bracket, and dental wires, the dishes, eyeglasses, sinks, kitchens, toilet bowls, toilets, bathtubs, bathrooms, washbowls, rest rooms, textiles, and clothing.

### Examples

To more closely examine the above embodiments of the antibacterial and antiviral agent according to the present invention, antibacterial and antiviral agents were prepared using various types of cyclodextrins, various ratios, and various compositions, and then the agents were evaluated.

### [Components and Composition of Agents Examined]

In Examples, agents that included the following components were examined and evaluated.

Octadecyl dimethyl(3-triethoxysilylpropyl)ammonium chloride (hereinafter referred to as Silicon-Containing Compound E or simply as Compound E), which is a silicon-containing compound (active ingredient that has an antibacterial and antiviral effect): 0.1 to 1.0% by weight
α-cyclodextrin (available under the trade name of "DEXYPEARL α-100" from Ensuiko Sugar Refining Co., Ltd.): 1 to 7% by weight
β-cyclodextrin (available under the trade name of "DEXYPEARL β-100" from Ensuiko Sugar Refining Co., Ltd.): 1% by weight
γ-cyclodextrin (available under the trade name of "DEXYPEARL γ-100" from Ensuiko Sugar Refining Co., Ltd.): 1 to 10% by weight
Complexed cyclodextrin (available under the trade name of "DEXYPEARL K-100" from Ensuiko Sugar Refining Co., Ltd.): 1 to 40% by weight
Branched cyclodextrin (available under the trade name of "ISOELEAT P" from Ensuiko Sugar Refining Co., Ltd., which includes maltosyl-α-cyclodextrin as a major component): 1 to 50% by weight
Solvent: Purified water

In the following description, drawings, and tables, "DEXYPEARL K-100" and "ISOELEAT P" are respectively referred to as "DEXYPEARL" and "ISOELEAT" for convenience.

### [Evaluation Items]

The following items were evaluated. Each of the items was described in detail below.
(1) Saturating concentration and change over time of various cyclodextrins
(2) Inclusion effect and change over time when the Silicon-Containing Compound E was included in (added to) the aqueous solutions of the various cyclodextrins at the saturating concentration according to (1)
(3) Qualitative evaluation and potentiometric titration of the various agents examined in (2)
(4) Deodorant activity test

Tables 1 to 5 summarize the results of various evaluations using the above respective cyclodextrins (CDs).

### <Solubility of CD>

"Solubility of CD" in Tables 1 to 5 shows whether a single CD as a solute was dissolved in purified water as a solvent at respective ratios shown in the respective tables (see "Ratio of CD Alone" in the tables). "Clarity" in "Solubility of CD" in the tables was determined by sequentially adding the CD and purified water at the respective ratio to a predetermined screw-cap bottle and stirring the mixture in the sealed bottle on a shaker to prepare the respective aqueous CD solution, which was then visually evaluated for clarity immediately after the preparation. When the CD aqueous solution was clear, the solution was rated as "OK". When the solution was not clear, the solution was rated as "NG". "Stability" in "Solubility of CD" in the tables was determined by allowing the respective aqueous CD solution after preparation to stand at ambient temperature for a predetermined period and then visually evaluating the conditions (such as presence of precipitates and deposits) of the solution. When the aqueous CD solution did not exhibit the conditions such as presence of precipitates and deposits, the solution was considered as stable (shown as "OK" in the tables). When the aqueous CD solution exhibited the conditions such as presence of precipitates and deposits, the solution was not considered as stable (shown as "NG" in the tables).

### <Solubility of Silicon-Containing Compound E>

To determine "Solubility of Compound E" in the tables, the Silicon-Containing Compound E as another solute was added at respective ratios shown in the respective tables (see "Ratio of Compound E" in the tables) to the respective aqueous CD solutions after evaluation of the solubility of CD, and then the mixture was stirred on a shaker to prepare the respective aqueous solutions of the CD and the Silicon-Containing Compound E. Immediately after preparation of the aqueous solutions of the CD and the Silicon-Containing Compound E, the clarity of the aqueous solutions was visually evaluated. When the aqueous solutions of the CD and the Silicon-Containing Compound E were clear, the solutions were rated as "OK". When the aqueous solutions of the CD and the Silicon-Containing Compound E were not clear, the solutions were rated as "NG". "Stability" in "Solubility of Compound E" in the tables was determined by allowing the respective aqueous solutions of the CD and the Silicon-Containing Compound E after preparation to stand at ambient temperature for a predetermined period and then visually evaluating the conditions (such as presence of precipitates and deposits) of the solutions. When the aqueous solutions of the CD and the Silicon-Containing Compound E did not exhibit the conditions such as presence of precipitates and deposits, the solutions were considered as stable (shown as "OK" in the tables). When the aqueous solutions of the CD and the Silicon-Containing Compound E exhibited the conditions such as presence of precipitates and deposits, the solutions were not considered as stable (shown as "NG" in the tables).

### <Evaluation of Functionality and Stability Over Time>

"Evaluations" in the respective tables shows the results of evaluation of the functionality (qualitative and quantitative evaluation) and evaluation of the stability over time (visual observation of change over time) of the respective aqueous solutions of the CD and the Silicon-Containing Compound E after evaluation of the solubility of the Silicon-Containing Compound E.

In the functionality evaluation, the qualitative evaluation was performed by applying a predetermined amount of the respective aqueous solution of the CD and the Silicon-Containing Compound E to a predetermined test specimen and detecting a change in pH (color change) with bromophenol blue to determine whether the Silicon-Containing Compound E was immobilized on the test specimen (see Fig.21). In particular, the qualitative evaluation was performed as follows. First, each of samples (an aqueous solution of 1% by weight of γ-CD and 1% by weight of the Compound E and an aqueous solution of 1% by weight of γ-CD and 0.1 % of the Compound E in the Example shown in Fig.21) was applied to a PP (polypropylene) film. After drying the sample, the film was washed with water. Then, a solution of 0.1% by weight of BPB (bromophenol blue) was sprayed onto the entire film. If the Silicon-Containing Compound E was immobilized on the surface of the PP film, a "blue color" appeared. In the Example shown in Fig.21, both of the two samples changed their color, compared with a film with no BPB solution applied (blank), which has confirmed that the Silicon-Containing Compound E was immobilized on the surface.

In the functionality evaluation, the quantitative evaluation was carried out by performing potentiometric titration of the samples (aqueous solutions) after evaluation of the stability over time, which is described below, (after storage test at 40°C for 3 months) to quantitatively determine the concentration (content) of the Compound E in the samples. In the potentiometric titration, predetermined electrodes were immersed in the samples, and titration was started with a titrant such as a diluted nitric acid solution, using a predetermined quantitative analyzer. During the titration, a curve of the electric potential over the samples against the volume of the titrant added was drawn to determine the inflection point in the curve, the point being taken as the end point. Fig.22 shows the results of measurement by the potentiometric titration. In the results of the potentiometric titration shown in Fig.22, α1E0 as a lot name, for example, means an aqueous solution that includes α-CD (1%), the Compound E as a reagent (a commercially available product, 0.1 %), and purified water (balance). Values shown in the table in Fig.22 indicate the concentration (%) of the Compound E in the respective lots after the storage test at 40°C for 3 months, as measured by the potentiometric titration. As an example of how to read the table in Fig.22, the lot "α1E01 (α-CD 1% + the Compound E 0.1%)" had an average concentration (%) of the Compound E of 0.07560 (%), the average concentration being obtained from three measurements by the potentiometric titration. The lot "α1E01 (α-CD 1% + the Compound E 0.1 %)" had a measurement (average) of 0.07560 (%), relative to the amount of the Compound E added of 0.1%. The quantitative results clearly show that the Compound E was detected in the aqueous Compound E solution of the respective lots, even after evaluation of the stability over time. The measurements were different from the theoretical value. The error may be caused by the variation in the purity of the Compound E reagents themselves and the uncertainty in preparation of the formulations.

The stability over time was evaluated by subjecting the respective aqueous solutions of the CD and the Silicon-Containing Compound E after evaluation of the solubility of the Silicon-Containing Compound E to an accelerated test (a three-month storage test at 40°C) and a cold test (a three-month storage test at about 5°C) and after the tests, visually observing a change in the condition (such as presence of precipitates and deposits) over time of the respective solutions.

### <Deodorant Activity Test>

The predetermined samples shown in Tables 1 to 5 were tested for deodorant activity against ammonia, acetic acid, isovaleric acid, and nonenal. Now, the test, including the test conditions, will be specifically described.

Test Items: The deodorant test and the test to gain SEK mark approval for textiles according to the certification standards of the Japan Textile Evaluation Technology Council

(4 ml of sample liquid was applied to a cotton fabric and dried for a period of from 30 minutes to 1 hour. Then, the concentration of the gas volatilized from the cotton fabric was measured by a detection tube and gas chromatography.)

Odor tested: Ammonia, acetic acid, isovaleric acid, and nonenal
Samples used: Sample 1): Compound E 0.1% + γ-CD 1% + purified water 98.9%
   Sample 2): Compound E 0.1% + γ-CD 5% + purified water 94.9%
Initial gas concentration: Ammonia: 100 ppm (100 cm²)
   Acetic acid: 50 ppm (100 cm²)
   Isovaleric acid: about 38 ppm (48 cm²)
   Nonenal: about 14 ppm (48 cm²)

As shown in Fig.23, the results of the deodorant activity test shows that the samples 1 and 2 exhibited a higher odor reduction (%) compared with a blank (a cotton fabric only) and had a great difference from the blank (a cotton fabric only), which has confirmed that the samples 1 and 2 could provide a deodorant effect.

**[Table 1]**

| | Solubility of CD | | | Solubility of Compound E | | | Evaluations | | |
|---|---|---|---|---|---|---|---|---|---|
| Type of CD | Ratio of CD Alone (w/w%) | Clarity | Stability | Ratio of Compound E (w/w%) | Clarity | Stability | Functionality (Immobilization) | | Change Over 3 Months (Accelerated Test at 40°C, Cold Test at 5°C, Ambient Test at 25°C) |
| | | | | | | | Qualitative | Quantitative* 1 | |
| α | 1 | OK | OK | 0.1 | NG | OK | Confirmed, though the solution reacted weakly | Quantitatively confirmed | Stable |
| | | | | 0.2 | NG | OK | | | Stable |
| | | | | 0.3 | NG | OK | | | Stable |
| | | | | 0.4 | NG | OK | | | Stable |
| | | | | 0.5 | NG | OK | Confirmed | | Stable |
| | | | | 0.6 | NG | OK | | | Stable |
| | | | | 0.7 | NG | OK | | | Stable |
| | | | | 0.8 | NG | NG | | | Clear but some precipitation was observed |
| | | | | 0.9 | NG | NG | | | Clear but some precipitation was observed |
| | | | | 1 | NG | NG | | | Clear but some precipitation was observed |
| | 2-6 | OK | OK | - | - | - | - | | - |
| | 7 | OK | OK | 0.1 | NG | OK | Confirmed | | Stable |
| | | | | 0.2 | NG | OK | | | Stable |
| | | | | 0.3 | NG | OK | | | Stable |
| | | | | 0.4 | NG | OK | | | Stable |
| | | | | 0.5 | NG | OK | | | Stable |
| | | | | 0.6 | NG | OK | | | Stable |
| | | | | 0.7 | NG | OK | | | Stable |
| | | | | 0.8 | NG | OK | | | Stable |
| | | | | 0.9 | NG | OK | | | Stable |
| | | | | 1 | NG | OK | | | Stable |
| | 8-10 | NG | NG | - | - | - | - | - | - |

| | | | | | | | | | |
|---|---|---|---|---|---|---|---|---|---|
| *1: 3 month storage test (accelerated condition: 40°C) -: The test was not performed. | | | | | | | | | |

**[Table 2]**

| | Solubility of CD | | | Solubility of Compound E | | | Evaluations | | |
|---|---|---|---|---|---|---|---|---|---|
| Type of CD | Ratio of CD Alone | Clarity | Stability | Ratio of Compound E (w/w%) | Clarity | Stability | Functionality (Immobilization) | | Change Over 3 Months (Accelerated Test at 40°C, Cold Test at 5°C, Ambient Test at 25°C) |
| | | | | | | | Qualitative | Quantitative* 1 | |
| | (w/w%) | | | | | | | | |
| | | | | 0.1 | NG | OK | Confirmed, though the solution reacted weakly | Quantitatively confirmed | Not changed |
| | | | | | | | | | |
| | | | | 0.2 | NG | OK | | - | Not changed |
| | | | | 0.3 | NG | OK | | | Not changed |
| | | | | 0.4 | NG | OK | | | Not changed |
| | 1 | OK | OK | 0.5 | NG | OK | Confirmed | | Not changed |
| β | | | | 0.6 | NG | OK | | | Not changed |
| | | | | 0.7 | NG | OK | | | Not changed |
| | | | | 0.8 | NG | OK | | | Not changed |
| | | | | 0.9 | NG | OK | | | Not changed |
| | | | | 1 | NG | OK | | | Not changed |
| | 2-10 | NG | NG | - | - | - | - | - | - |

| | | | | | | | | | |
|---|---|---|---|---|---|---|---|---|---|
| * 1: 3 month storage test (accelerated condition: 40°C) -: The test was not performed. | | | | | | | | | |

**[Table 3]**

| | Solubility of CD | | | Solubility of Compound E | | | Evaluations | | | Deodorant Effect |
|---|---|---|---|---|---|---|---|---|---|---|
| Type of | Ratio of | Clarity | Stability | Ratio of | Clarity | Stability | Functionality | | Change Over | Deodorant |
| CD | CD Alone (w/w%) | | | Compound E (w/w%) | | | (Immobilization) | | 3 Months (Accelerated Test at 40°C, Cold Test at 5°C, Ambient Test at 25°C) | Activity Test (by Detection Tube and Gas Chromatography) |
| | | | | | | | Qualitative | Quantitative* 1 | | |
| γ | 1 | OK | OK | 0.1 | OK | OK | Confirmed, though the solution reacted weakly | Quantitatively confirmed | Not changed | Confirmed |
| | | | | 0.2 | OK | OK | | - | Not changed | - |
| | | | | 0.3 | OK | OK | | Quantitatively confirmed | Not changed | |
| | | | | 0.4 | OK | OK | | - | Not changed | |
| | | | | 0.5 | NG | OK | Confirmed | Quantitatively confirmed | Not changed | |
| | | | | 0.6 | NG | OK | | - | Some | |
| | | | | | | | | | precipitation was observed | |
| | | | | 0.7 | NG | OK | | Quantitatively confirmed | Some precipitation was observed | |
| | | | | 0.8 | NG | OK | | - | Some precipitation was observed | |
| | | | | 0.9 | NG | OK | | - | Some precipitation was observed | |
| | | | | 1 | NG | OK | | Quantitatively confirmed | Some precipitation was observed | |
| | 2 | OK | OK | - | - | - | - | - | - | |
| | 3 | OK | OK | 0.1 | NG | OK | Confirmed, though the solution reacted weakly | Quantitatively confirmed | Not changed | |
| | | | | 0.2 | NG | OK | | - | Not changed | |
| | | | | 0.3 | NG | OK | | | Not changed | |
| | | | | 0.4 | NG | OK | | | Not changed | |
| | | | | 0.5 | NG | OK | Confirmed | | Not changed | |
| | | | | 0.6 | NG | OK | | | Not changed | |
| | | | | 0.7 | NG | OK | | | Not changed | |
| | | | | 0.8 | NG | OK | | | Not changed | |
| | | | | 0.9 | NG | OK | | | Not changed | |
| | | | | 1 | NG | OK | | | Not changed | |
| | 4-5 | OK | OK | - | - | - | - | - | - | Confirmed in case of γ-CD 5% + Etak 0.1% |
| | 6 | OK | OK | 0.1 | NG | OK | Confirmed, though the solution reacted weakly | Quantitatively confirmed | Not changed | |
| | | | | 0.2 | NG | OK | | - | Not changed | |
| | | | | 0.3 | NG | OK | | | Not changed | |
| | | | | 0.4 | NG | OK | | | Not changed | |
| | | | | 0.5 | NG | OK | Confirmed | | Not changed | |
| | | | | 0.6 | NG | OK | | | Not changed | |
| | | | | 0.7 | NG | OK | | | Not changed | |
| | | | | 0.8 | NG | OK | | | Not changed | |
| | | | | 0.9 | NG | OK | | | Not changed | |
| | | | | 1 | NG | OK | | | Not changed | |
| | 7-9 | OK | OK | - | - | - | - | - | - | |
| | 10 | OK | OK | 0.1 | NG | NG | Confirmed, though the solution reacted weakly | Quantitatively confirmed | Precipitated | |
| | | | | 0.2 | NG | NG | | | Precipitated | |
| | | | | 0.3 | NG | NG | | | Precipitated | |
| | | | | 0.4 | NG | NG | | | Precipitated | |
| | | | | 0.5 | NG | NG | Confirmed | | Precipitated | |
| | | | | 0.6 | NG | NG | | | Precipitated | |
| | | | | 0.7 | NG | NG | | | Precipitated | |
| | | | | 0.8 | NG | NG | | | Precipitated | |
| | | | | 0.9 | NG | NG | | | Precipitated | |
| | | | | 1 | NG | NG | | | Precipitated | |
| | 15 | NG | OK | - | - | - | - | - | - | |
| | 20 | NG | OK | - | - | - | - | - | - | |
| | 25 | NG | NG | - | - | - | - | - | - | |

| | | | | | | | | | | |
|---|---|---|---|---|---|---|---|---|---|---|
| * 1: 3 month storage test (accelerated condition: 40°C) -: The test was not performed. | | | | | | | | | | |

**[Table 4]**

| | Solubility of CD | | | Solubility of Compound E | | | Evaluations | | |
|---|---|---|---|---|---|---|---|---|---|
| Type of CD | Ratio of CD Alone (w/w%) | Clarity | Stability | Ratio of Compound E (w/w%) | Clarity | Stability | Functionality (Immobilization) | | Change Over 3 Months (Accelerated Test at 40°C, Cold Test at 5°C, Ambient Test at 25°C) |
| | | | | | | | Qualitative | Quantitative* 1 | |
| Branched (ISOELEAT) | 1 | OK | OK | 0.1 | OK | OK | OK | Quantitatively confirmed | Not clear but stable |
| | 2 | OK | OK | 0.1 | OK | OK | OK | - | Not clear but stable |
| | 3 | OK | OK | 0.1 | OK | OK | OK | | Not clear but stable |
| | 4 | OK | OK | 0.1 | OK | OK | OK | | Not clear but stable |
| | 5 | OK | OK | 0.1 | OK | OK | OK | | Not clear but stable |
| | 6 | OK | OK | 0.1 | OK | OK | OK | | Not clear but stable |
| | 7 | OK | OK | 0.1 | OK | OK | OK | | Not clear but stable |
| | 8 | OK | OK | 0.1 | OK | OK | OK | | Not clear but stable |
| | 9 | OK | OK | 0.1 | OK | OK | OK | | Not clear but stable |
| | 10 | OK | OK | 0.1 | NG | NG | - | | Precipitated |
| | 20 | OK | OK | 0.1 | NG | NG | - | | Precipitated |
| | 30 | OK | OK | 0.1 | NG | NG | - | | Precipitated |
| | 40 | OK | OK | 0.1 | NG | NG | - | | Precipitated |
| | 50 | OK | OK | 0.1 | NG | NG | - | | Precipitated |

| | | | | | | | | | |
|---|---|---|---|---|---|---|---|---|---|
| * 1: 3 month storage test (accelerated condition: 40°C) -: The test was not performed. | | | | | | | | | |

**[Table 5]**

| | Solubility of CD | | | Solubility of Compound E | | | Evaluations | | |
|---|---|---|---|---|---|---|---|---|---|
| Type of CD | Ratio of CD Alone | Clarity | Stability | Ratio of Compound E (w/w%) | Clarity | Stability | Functionality (Immobilization) | | Change Over 3 Months (Accelerated Test at 40°C, Cold Test at 5°C, Ambient Test at 25°C) |
| | | | | | | | Qualitative | Quantitative* 1 | |
| | (w/w%) | | | | | | | | |
| | 1 | OK | OK | 0.1 | OK | OK | OK | Quantitatively confirmed | Not clear but stable |
| | 2 | OK | OK | 0.1 | OK | OK | OK | - | Not clear but stable |
| | 3 | OK | OK | 0.1 | OK | OK | OK | | Not clear but stable |
| | 4 | OK | OK | 0.1 | OK | OK | OK | | Not clear but stable |
| | 5 | OK | OK | 0.1 | OK | OK | OK | | Not clear but stable |
| | 6 | OK | OK | 0.1 | OK | OK | OK | | Not clear but stable |
| | 7 | OK | OK | 0.1 | OK | OK | OK | | Not clear but stable |
| | 8 | OK | OK | 0.1 | OK | OK | OK | | Not clear but stable |
| | 9 | OK | OK | 0.1 | OK | OK | OK | | Not clear but stable |
| | 10 | OK | OK | 0.1 | OK | OK | OK | | Not clear but stable |
| | 20 | OK | OK | 0.1 | OK | NG | - | | Precipitated |
| | 30 | OK | OK | 0.1 | NG | NG | - | | Precipitated |
| | 40 | OK | OK | 0.1 | NG | NG | - | | Precipitated |
| | 50 | NG | NG | - | - | - | - | | - |

| | | | | | | | | | |
|---|---|---|---|---|---|---|---|---|---|
| * 1: 3 month storage test (accelerated condition: 40°C) -: The test was not performed. | | | | | | | | | |

Fig. illustrates the condition of the aqueous solutions of the CD and the Silicon-Containing Compound E that have a "Ratio of CD Alone" of 1% and a "Ratio of Silicon-Containing Compound E" of from 0.1% by weight to 1.0% by weight as illustrated in Table 3. The "Ratio of Silicon-Containing Compound E" increases by 0.1% from left to right. As illustrated in Fig.1, the aqueous solutions of the CD and the Silicon-Containing Compound E ("Ratio of CD Alone": 1% by weight) that have a "Ratio of Compound E" of from 0.1 to 0.4% by weight were relatively clear (rated as OK in Table 1), while the aqueous solutions that has a "Ratio of Compound E" of from 0.5 to 1.0% by weight were cloudy (rated as NG in Table 3).

Fig.2 illustrates the aqueous solutions of the CD and the Silicon-Containing Compound E that have a "Ratio of CD Alone" of 6% and a "Ratio of Compound E" of from 0.1% by weight to 0.9% by weight as illustrated in Table 3. The "Ratio of Silicon-Containing Compound E" sequentially increases by 0.1%.

Fig.3 illustrates the aqueous solutions of the CD and the Silicon-Containing Compound E that have a "Ratio of CD Alone" of 7% and a "Ratio of Compound E" of from 0.1% by weight to 1.0% by weight as illustrated in Table 3. The "Ratio of Compound E" sequentially increases by 0.1%.

Fig.4 illustrates the aqueous solutions of the CD and the Silicon-Containing Compound E that have a "Ratio of CD Alone" of 10% and a "Ratio of Compound E" of from 0.1% by weight to 1.0% by weight as illustrated in Table 3. The "Ratio of Compound E" sequentially increases by 0.1%. In Fig.4, all of the aqueous solutions of the CD and the Silicon-Containing Compound E ("Ratio of CD Alone": 10% by weight) that have a "Ratio of Compound E" of from 0.1 to 1.0% by weight have formed white precipitates (rated as NG in Table 3).

Figs.9 to 16 illustrate "Change Over 3 Months (storage test at 40°C)" of "Evaluations" in the Table 3 (Type of CD: γ-cyclodextrin).

(Summary of Results of Evaluations of Aqueous Solutions of γ-CD and Silicon-Containing Compound E)

In Table 3, "Solubility of Compound E" shows that the solutions that included γ-cyclodextrin in an amount of 1% by weight and the Silicon-Containing Compound E in an amount equal to or less than 0.4% by weight were clear. The solutions that included γ-cyclodextrin in an amount of 1% by weight and the Silicon-Containing Compound E in an amount equal to or more than 0.5% by weight were not clear, while the conditions of the solutions were stable. The evaluation of the stability over time has shown that the solutions that included γ-cyclodextrin in an amount equal to or less than 0.6% by weight were not essentially changed. And it has been confirmed that the solutions that included γ-cyclodextrin in an amount equal to or more than 10% by weight formed precipitates of the γ-cyclodextrin together with precipitates of the Silicon-Containing Compound E. The results have led to the conclusion that γ-cyclodextrin is preferably included in an amount equal to or more than 1% by weight and equal to or less than 6% by weight, and the Silicon-Containing Compound E is preferably included in an amount equal to or less than 1% by weight to ensure stability over time, because of the product requirement that the aqueous solution of the Silicon-Containing Compound E have stability over time. The clarity is an additional design option. If it is necessary that the solution as a product be clarity, the ratio of the γ-cyclodextrin may be appropriately adjusted to achieve clarity as appropriate.

Although a case in which γ-cyclodextrin is included in an amount less than 1% by weight was not evaluated, such amount of γ-cyclodextrin dissolves well. Thus, it is estimated that the effects of the present invention are produced in response to the amount of γ-cyclodextrin used.

And the Silicon-Containing Compound E and γ-cyclodextrin are preferably included at a weight ratio of from 0.1:6 to 1:1 (the silicon-containing compound: γ-cyclodextrin).

Figs.5 to 8 illustrate "Change Over 3 Months (storage test at 40°C)" of "Evaluations" in the Table 1 (Type of CD: α-cyclodextrin).

### (Summary of Results of Evaluations of Aqueous Solution of α-CD and Silicon-Containing Compound E)

In Table 1, "Solubility of Compound E" shows that all of the solutions were not clarity, while the solutions that included α-cyclodextrin in an amount of 1% by weight and the Silicon-Containing Compound E in an amount equal to or less than 0.7% by weight and the solutions that included α-cyclodextrin in an amount of 7% by weight and the Silicon-Containing Compound E in an amount of from 0.1 to 1.0% by weight remained stable as a solution. The evaluation of the stability over time has shown that the solutions that included α-cyclodextrin in an amount equal to or less than 1% by weight and α-cyclodextrin in an amount less than 0.7% by weight and the solutions that included α-cyclodextrin in an amount of 7% by weight and α-cyclodextrin in an amount of from 0.1 to 0.7% by weight were stable. The results have led to the conclusion that α-cyclodextrin is preferably included in an amount equal to or less than 7% by weight, and the Silicon-Containing Compound E is preferably included in an amount equal to or less than 1% by weight (more preferably in an amount equal to or less than 0.7% by weight) to ensure stability over time, because of the product requirement that the aqueous solution of the Silicon-Containing Compound E have stability over time.

Although a case in which α-cyclodextrin is included in an amount less than 1% by weight was not evaluated, such amount of α-cyclodextrin dissolves well. Thus, it is estimated that the effects of the present invention are produced in response to the amount of α-cyclodextrin used.

And the Silicon-Containing Compound E and α-cyclodextrin are preferably included at a weight ratio of from 0.1:7 to 1:1 (the silicon-containing compound: α-cyclodextrin).

### (Summary of Results of Evaluations of Aqueous Solution of β-CD and Silicon-Containing Compound E)

In Table 2, "Solubility of Compound E" shows that the solutions that included the Silicon-Containing Compound E in any of the amounts were not clear but remained stable as a solution. The evaluation of the stability over time has shown that all of the solutions were not changed. The results have led to the conclusion that α-cyclodextrin is preferably included in an amount of 1% by weight, and the Silicon-Containing Compound E is preferably included in an amount of from 0.1 to 1% by weight inclusive to ensure stability over time, because of the product requirement that the aqueous solution of the Silicon-Containing Compound E have stability over time.

Although a case in which β-cyclodextrin is included in an amount less than 1% by weight was not evaluated, such amount of β-cyclodextrin dissolves well. Thus, it is estimated that the effects of the present invention are produced in response to the amount of β-cyclodextrin used.

And the Silicon-Containing Compound E and β-cyclodextrin are preferably included at a weight ratio of from 0.1:1 to 1:1 (the silicon-containing compound: β-cyclodextrin).

Figs. 17 and 20 illustrate "Change Over 3 Months (storage test at 40°C)" of "Evaluations" in the Table 4 (Type of CD: a branched cyclodextrin (ISOELEAT P)).

### (Summary of Results of Evaluations of Aqueous Solutions of ISOELEAT and Silicon-Containing Compound E)

In Table 4, "Solubility of Compound E" shows that the solutions that included ISOELEAT in an amount of from 1 to 9% by weight and the Silicon-Containing Compound E in an amount of 0.1% by weight remained clear. And the solutions that included ISOELEAT in an amount equal to or less than 9% by weight remained stable as a solution. The evaluation of the stability over time has shown that the solutions that included ISOELEAT in an amount equal to or less than 9% by weight were stable. And it has been confirmed that the solutions that included ISOELEAT in an amount equal to or more than 10% by weight formed precipitates of the ISOELEAT together with precipitates of the Silicon-Containing Compound E. The results have led to the conclusion that the solutions that include the Silicon-Containing Compound E in an amount of 0.1% by weight preferably include ISOELEAT in an amount of from 1 to 9% by weight to ensure stability over time, because of the product requirement that the aqueous solution of the Silicon-Containing Compound E have stability over time. The evaluation of the stability over time has shown that the aqueous solutions were not clear but stable as a formulation. Although a case in which ISOELEAT is included in an amount less than 1% by weight was not evaluated, such amount of ISOELEAT dissolves well. Thus, it is estimated that the effects of the present invention are produced in response to the amount of ISOELEAT used.

And the Silicon-Containing Compound E and ISOELEAT are preferably included at a weight ratio of from 0.1:9 to 0.1:1 (the silicon-containing compound: ISOELEAT).

Figs. 18 to 20 illustrate "Change Over 3 Months (storage test at 40°C)" of "Evaluations" in the Table 5 (Type of CD: a complexed cyclodextrin (DEXYPEARL K-100)).

### (Summary of Results of Evaluations of Aqueous Solutions of DEXYPEARL and Silicon-Containing Compound E)

In Table 5, "Solubility of Compound E" shows that the solutions that included DEXYPEARL in an amount of from 1 to 10% by weight and the Silicon-Containing Compound E in an amount of 0.1% by weight remained clear. The solutions that included DEXYPEARL in an amount equal to or less than 10% by weight were stable as a solution. The evaluation of the stability over time has shown that the solutions that included DEXYPEARL in an amount equal to or less than 10% by weight were stable, while the solutions that included DEXYPEARL in an amount equal to or more than 20% by weight formed precipitates of the DEXYPEARL together with precipitates of the Silicon-Containing Compound E. The results have led to the conclusion that the solutions that include the Silicon-Containing Compound E in an amount of 0.1% by weight preferably include DEXYPEARL in an amount of from 1 to 10% by weight to ensure stability over time, because of the product requirement that the aqueous solution of the Silicon-Containing Compound E have stability over time. The evaluation of the stability over time has shown that the aqueous solutions were not clear but stable as a formulation.

Although a case in which DEXYPEARL is included in an amount less than 1% by weight was not evaluated, such amount of DEXYPEARL dissolves well. Thus, it is estimated that the effects of the present invention are produced in response to the amount of DEXYPEARL used.

And the Silicon-Containing Compound E and DEXYPEARL are preferably included at a weight ratio of from 0.1:10 to 0.1:1 (the silicon-containing compound: DEXYPEARL).

### [Summary of Results of Evaluations]

Now, the results of evaluations of cyclodextrins (α-cyclodextrin, β-cyclodextrin, the complexed cyclodextrin, and the branched cyclodextrin) that were performed in a manner similar to the manner used for the γ-cyclodextrin, as well as the results of evaluations of γ-cyclodextrin described above will be summarized.
(1) It has been confirmed that the included conditions, the preferred amounts, and the stability over time of the Silicon-Containing Compound E varied depending on the type of cyclodextrin.
(2) Even when the solutions were not clear (cloudy), the solutions stably included the Silicon-Containing Compound E unless the solutions were not separated (which was confirmed by the qualitative evaluation).
(3) The solutions that included α-cyclodextrin in an amount of 1% by weight and the Silicon-Containing Compound E in an amount of from 0.1 to 0.7% by weight were stable over time. The solutions that included α-cyclodextrin in an amount of 7% by weight and the Silicon-Containing Compound E in an amount of from 0.1 to 1.0% by weight were stable.
(4) The solutions that included β-cyclodextrin in an amount of 1% by weight and the Silicon-Containing Compound E in an amount of from 0.1 to 1.0% were stable over time.
(5) The solutions that included γ-cyclodextrin in an amount equal to or less than 6% by weight and the Silicon-Containing Compound E in an amount of from 0.1 to 1.0% by weight were stable over time. The solutions that included γ-cyclodextrin in an amount of 10% by weight were not stable over time.
(6) The solutions that included ISOELEAT, which is a branched CD, in an amount equal to or less than 9% by weight and the Silicon-Containing Compound E in an amount of 0.1% by weight were stable over time.
(7) The solutions that included DEXYPEARL, which is a complexed CD, in an amount equal to or less than 6% by weight and the Silicon-Containing Compound E in an amount of 0.1% by weight were stable over time.

### [Evaluation of Antibacterial Properties]

Next, an antibacterial and antiviral agent prepared with exemplary composition according to the Examples (an aqueous solution of 1% by weight of γ-CD and 0.1% by weight of the Compound E) was evaluated for antibacterial properties. Now, a test method for evaluating the antibacterial properties of the antibacterial and antiviral agent prepared with the composition and the results of the test will be described.

Test method: (1) Three culture petri dishes (with a diameter of 90 mm) were prepared. To two of the dishes, 1 ml of the antibacterial and antiviral agent with the composition (an aqueous solution of 1% by weight of γ-CD and 0.1% by weight of the Compound E) was added to prepare two samples for evaluation, which was then dried in a safety cabinet.

(2) After drying, one of the samples for evaluation was washed with saline in the petri dish to remove impurities and the unattached Compound E. To compare with the two samples for evaluation, a petri dish with no antibacterial and antiviral agent with the above composition added was used as a blank.

(3) A bacteria (MRSA) suspension was prepared in normal liquid medium.

(4) The bacteria suspension prepared (3.50 × 10⁴/0.5 ml) was added to each of the dishes and allowed to stand for 30 minutes.

(5) After 30 minutes, nutrient agar medium was added to each of the dishes and then cultured by pour plating (for an hour).

The three dishes: the untreated dish with no antibacterial and antiviral agent prepared with the above composition (referred to as "blank (untreated)"), the dish that underwent antibacterial treatment by addition of the antibacterial and antiviral agent prepared with the above composition as a sample for evaluation (referred to as "sample treated with an aqueous solution of the Compound E"), and the dish that underwent antibacterial treatment by addition of the antibacterial and antiviral agent prepared with the above composition and then was washed with water (referred to as "sample treated with an aqueous solution of the Compound E and washed with water") were evaluated for antibacterial properties.

The test results have shown that the dishes on which the antibacterial and antiviral agent prepared with the above composition was immobilized (illustrated as "Treatment with Aqueous Solution of Compound E (white cube)" or "Treatment with Aqueous solution of Compound E + Washing with Water (black triangle)" in Fig.24) exhibited a reduction in the number of bacteria, compared with the blank dish (illustrated as "Blank (Untreated) (black diamond)" in Fig.21). And it has been confirmed that the results of the sample dishes were significant with respect to the result of the blank dish. Thus, the antibacterial and antiviral agent prepared with the above composition has been demonstrated to have an antibacterial effect.

As described above, in the present invention, inclusion of a silicon-containing compound itself as a guest molecule in a cyclodextrin or a cyclodextrin derivative allows dissolution of the silicon-containing compound and provision of an antibacterial and antiviral agent that includes an aqueous solution that has high stability over time. Thus, an object where use of an organic solvent such as an alcohol is avoided can be rendered antibacterial and antiviral. The present invention can also provide a deodorant effect through the cyclodextrin or the cyclodextrin derivative included in the antibacterial and antiviral agent.

Conventionally, an alcohol has been included to dissolve a silicon-containing compound that has an antibacterial and antiviral effect. In the present invention, inclusion of a silicon-containing compound itself as a guest molecule in a cyclodextrin or a cyclodextrin derivative allows dissolution of the silicon-containing compound in water to form an aqueous solution. This can allows immobilization of the antibacterial ingredient on the target surface, which is a characteristic of the antibacterial and antiviral agent according to the present invention, and provision of a deodorant effect through the cyclodextrin.

### Industrial Applicability

The antibacterial and antiviral agent according to the present invention can render an object where use of an organic solvent such as an alcohol is avoided antibacterial and antiviral, and thus the agent is industrially useful.

## Claims

1. An aqueous solution of a silicon-containing compound, the solution comprising a silicon-containing compound and a cyclodextrin or a cyclodextrin derivative.

2. The aqueous solution of a silicon-containing compound according to claim 1, wherein the silicon-containing compound is represented by the following general formula (1): 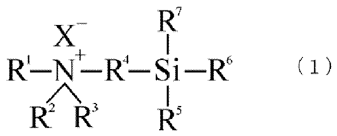 wherein R1 represents a hydrocarbon group having 6 or more carbon atoms, R2 and R3 are optionally the same or different and represent a lower hydrocarbon group, R4 represents a divalent lower hydrocarbon group, R5, R6, and R7 are optionally the same or different and represent a lower alkyl group or a lower alkoxy group, and X represents a halogen ion or an organic carbonyloxy ion.

3. The aqueous solution of a silicon-containing compound according to claim 1 or 2,
wherein the aqueous solution comprises the silicon-containing compound in an amount equal to or more than 0.001 % by weight.

4. The aqueous solution of a silicon-containing compound according to any one of claims 1 to 3,
wherein the aqueous solution comprises the silicon-containing compound and the cyclodextrin or the cyclodextrin derivative at a weight ratio of from 0.1:30 to 10:1.

5. The solution of a silicon-containing compound according to any one of claims 1 to 4,
wherein the cyclodextrin or the cyclodextrin derivative is at least one selected from the group consisting of α-cyclodextrin, β-cyclodextrin, γ-cyclodextrin, branched cyclodextrins, and complexed cyclodextrins.

6. The aqueous solution of a silicon-containing compound according to any one of claims 1 to 5,
wherein the silicon-containing compound represented by the general formula (1) comprises octadecyl dimethyl(3-triethoxysilylpropyl)ammonium chloride.

7. An antibacterial and antiviral agent comprising the aqueous solution of a silicon-containing compound according to any one of claims 1 to 6.
